# FASCICULE DE BREVET EUROPEEN

(11) **EP 2 361 241 B1**
(45) Date de publication et mention de la délivrance du brevet: **18.09.2013**
(21) Numéro de dépôt: 09768202.5
(22) Date de dépôt: 17.11.2009
(51) Int. Cl.: C07C 229/08, C07C 227/04

(54) **PROCEDE DE SYNTHESE D'UN OMEGA-AMINOACIDE OU ESTER A PARTIR D'UN ACIDE OU ESTER GRAS MONO-INSATURE**
VERFAHREN ZUR SYNTHESE EINER OMEGA-AMINOSÄURE ODER EINES OMEGA-AMINOSÄUREESTERS AUSGEHEND VON EINER EINFACH UNGESÄTTIGTEN FETTSÄURE BZW. EINEM EINFACH UNGESÄTTIGTEN ESTER
METHOD FOR THE SYNTHESIS OF AN OMEGA-AMINO ACID OR ESTER STARTING FROM A MONOUNSATURATED FATTY ACID OR ESTER

(30) Priorité: 17.11.2008 FR 0857780
(43) Date de publication de la demande: 31.08.2011
(73) Titulaire: Arkema France, 92700 Colombes (FR)
(72) Inventeur: DUBOIS, Jean-Luc, F-69390 Millery (FR)
(86) Numéro de dépôt international: PCT/FR2009/052196
(87) Numéro de publication internationale: WO 2010/055273

(56) Documents cités:
- WO-A-03/093215
- WO-A-2005/026106
- WO-A-2008/104722
- COTARCA L ET AL: "Efficient and scaleable methods for omega-functionalized nonanoic acids" ORGANIC PROCESS RESEARCH AND DEVELOPMENT, CAMBRIDGE, GB, vol. 5, no. 1, 1 janvier 2001 (2001-01-01) , pages 69-76, XP002459983

## Description

L'invention vise un procédé de synthèse d'acides ω-amino-alcanoïques ou de leurs esters à partir d'acides gras naturels insaturés transitant par un composé intermédiaire de type nitrile ω-insaturé.
L'industrie des polyamides utilise toute une gamme de monomères constitués par des ω-amino-acides à longue chaîne, usuellement appelés Nylon, caractérisés par la longueur de chaîne méthylène (-CH₂)ₙ séparant deux fonctions amide -CO-NH-, C'est ainsi que sont connus les Nylon-6, Nylon 6-6, Nylon 6-10, Nylon 7, Nylon 8, Nylon 9, Nylon 11, Nylon 13, etc...
Ces monomères sont par exemple fabriqués par voie de synthèse chimique en utilisant notamment comme matière première des oléfines en C2 à C4, des cycloalcanes ou du benzène, mais aussi de l'huile de ricin (Nylon 11), de l'huile érucique ou lesquérolique (Nylon 13)...
L'évolution actuelle en matière d'environnement conduit dans les domaines de l'énergie et de la chimie à privilégier l'exploitation des matières premières naturelles provenant d'une source renouvelable. C'est la raison pour laquelle certains travaux ont été repris pour élaborer sur le plan industriel des procédés utilisant des acides/esters gras comme matière première de fabrication de ces monomères.
Ce type d'approche n'a que peu d'exemples industriels. Un des rares exemples de procédé industriel utilisant un acide gras comme matière première est celui de la fabrication à partir de l'acide ricinoléique extrait de l'huile de ricin, de l'acide amino-11-undécanoique, qui est à la base de la synthèse du Rilsan 11 ^{®}. Ce procédé est décrit dans l'ouvrage « Les Procédés de Pétrochimie » de A. Chauvel et al. paru aux Editions TECHNIP (1986). L'acide amino-11-undécanoique est obtenu en plusieurs étapes. La première consiste en une méthanolyse de l'huile de ricin en milieu basique produisant le ricinoléate de méthyle qui est ensuite soumis à une pyrolyse pour obtenir, d'une part l'heptanaldéhyde et, d'autre part le undécylénate de méthyle. Ce dernier est passé en forme acide par hydrolyse. Ensuite l'acide formé est soumis à une hydrobromuration pour donner l'acide ω-bromé d'où l'on passe par amination à l'acide amino-11-undécanoïque.
Les principaux travaux ont porté sur la synthèse de l'acide amino-9-nonanoïque qui est le précurseur du Nylon 9 à partir de l'acide oléique d'origine naturelle. En ce qui concerne ce monomère particulier on peut citer *l'ouvrage* « n-Nylons, Their Synthesis, Structure and Properties » - 1997 Ed.J. Wiley et Sons dont le chapitre 2.9 (pages 381 à 389) est consacré au 9-Nylon. Cet article donne la synthèse des réalisations et des travaux conduits sur le sujet. On y mentionne, page 381, le procédé développé par l'ex Union Soviétique ayant conduit à la commercialisation du Pelargon®. On y mentionne, page 384, également un procédé développé au Japon utilisant l'acide oléique venant de l'huile de soja comme matière première. La description correspondante fait référence à l'ouvrage de A. Ravve « Organic Chemistry of Macromolecules » (1967) Marcel Dekker, Inc., dont la partie 15 est consacrée aux polyamides et qui mentionne en page 279 l'existence d'un tel procédé.
Pour être complet sur l'état de l'art en la matière, il faut citer les nombreux articles publiés par E. H. Pryde et al. entre 1962 et 1975 dans - Journal of the American Oil Chemists ' Society - « Aldehydic Materials by the Ozonization of Vegetable Oils » Vol. 39 pages 496-500 ; « Pilot Run, Plant Design and Cost Analysis for Reductive Ozonolysis of Methyl Soyate » Vol. 49 pages 643-648 et R.B. Perkins et al. « Nylon-9 from Unsaturated Fatty Derivatives : Preparation and Characterization » JAOCS, Vol. 52 pages 473-477. Il est à noter que le premier de ces articles fait aussi référence page 498 à des travaux antérieurs réalisés par des Japonais : H. Otsukl et H. Funahashi.
Pour résumer cette partie de l'état de l'art visant ce type de synthèse du « Nylon 9 » à partir d'huiles végétales on peut décrire le mécanisme réactionnel simplifié suivant appliqué à l'ester oléique, extrait des huiles par méthanolyse : Ozonolyse réductrice

H₃C-(CH₂)₇-CH=CH-(CH₂)₇-COOCH₃ + (O₃, H₂) → HOC-(CH₂)₇-COOCH₃ + H₃C-(CH₂)₇-COH

### Amination réductrice

HOC-(CH₂)₇-COOCH₃ + (NH₃, H₂) → H₂N-(CH₂)₈-COOCH₃ + H₂O

### Hydrolyse

HOC-(CH₂)₈-COOCH₃ +H₂O → H₂N-(CH₂)₈-COOH + CH₃OH

Cette voie très séduisante sur le plan réactionnel présenté cependant un inconvénient économique important constitué par la production lors de la première étape d'un aldéhyde à longue chaîne (9 atomes de carbone au total) pratiquement pas valorisable notamment dans l'industrie des polymères de type polyamides.
Le brevet UK n° 741,739 décrit pour sa part la synthèse de ce même acide à partir de l'acide oléique mais en utilisant la voie oléonitrile. Le schéma réactionnel simplifié de ce procédé est le suivant. Une voie analogue est citée dans l'article de R.B. Perkins et al cité ci-dessus p. 475.

H₃C-(CH₂)₇-CH=CH-(CH₂)₇-COOH + NH₃ → H₃C-(CH₂)₇-CH=CH-(CH₂)₇-CN + 2 H₂O

H₃C-(CH₂)₇-CH=CH-(CH₂)₇-CN + (O₃) + H₂O) → H₃C-(CH₂)₇-COOH + CN-(CH₂)₇-COOH

CN-(CH₂)₇-COOH + 2 H₂ → H₂N-(CH₂)₈-COOH

Cette synthèse conduit à l'acide pélargonique H₃C-(CH₂)₇-COOH comme sous-produit.
Plus récemment, ont été développés des procédés de fabrication de monomères ω-aminoacides qui utilisent notamment une réaction de métathèse.
On peut citer à ce sujet le document de brevet WO03093215 qui décrit la métathèse d'acides ou d'esters gras insaturés avec une oléfine, telle que l'éthylène, suivie d'amination de l'acide ou ester insaturé obtenu conduisant à un α,ω-aminoacide.
La demanderesse a pour sa part développé un procédé dans lequel la fonction amine terminale de l'ω-aminoacide résulte de l'hydrogénation d'une fonction nitrile introduite par métathèse croisée d'acides ou d'esters gras naturels avec l'acrylonitrile. Ce procédé est décrit dans le document de brevet WO02008104722.
La présente invention vise à proposer un nouveau procédé pour synthétiser toute une gamme d'acides ω-amino-alcanoïques ou de leurs esters à partir d'acides gras naturels insaturés.
Le problème est donc de trouver un procédé de synthèse de divers ω-amino-acides de formule H₂N-(CH₂)ₙ-COOH (et de leurs polymères) dans laquelle n est compris entre 3 et 14 à partir de matières premières renouvelables (de très large accès et donc peu onéreuses), simple à mettre en oeuvre tout en évitant d'une part les contraintes environnementales évoquées précédemment et d'autre part les handicaps économiques dus aux sous-produits des réactions.

La solution proposée consiste à travailler à partir de matières premières constituées par des acides gras insaturés à longue chaîne naturels, à les transformer dans un premier stade en nitriles ω-insaturés puis ensuite dans un deuxième stade à « réinsérer » une fonction acide carboxylique dans le composé par action sur la double liaison terminale du nitrile ω-insaturé, soit au moyen d'une coupure oxydante, soit par réaction de métathèse croisée avec un composé de type acrylate.

On entend par acide gras naturel un acide issu des milieux végétal ou animal, y compris les algues, plus généralement du règne végétal, et donc renouvelable. Cet acide comportera au moins une insaturation oléfinique dont la localisation en position x par rapport au groupement acide (delta x) et comportant au moins 10 et de préférence au moins 14 atomes de carbone par molécule permettra de déterminer la formule de l'ω-amino-acide final.
On peut citer à titre d'exemples de tels acides, les acides en C10, les acides obtusilique (cis-4-décénoïque) et caproléique (cis-9-décénoïque), les acides en C12, les acides lauroléique (cis-5-dodécénoïque) et lindérique (cis-4-dodécénoïque), les acides en C14, les acides myristoléique (cis-9-tétradécénoïque), physétérique (cis-5-tetradécénoïque) et tsuzuique (cis-4-tetradécénoïque), l'acide en C16, l'acide palmitoléique (cis-9- hexadécénoïque), les acides en C18, les acides oléique (cis-9-octadécénoïque), élaidique (trans-9-octadécénoïque), pétrosélinique (cis-6-octadécénoïque), vaccénique (cis-11-octadécénoïque) et ricinoléique (12-hydroxy--cis-9-octadécénoïque), les acides en C20, les acides gadoléique (cis-9-eicosénoïque), gondoïque (cis-11-eicosénoïque), l'acide cis-5-eicosènoïque et l'acide lesquérolique (14-hydroxy-cis-11-eicosénoïque), les acides en C22, les acides cétoléique (cis-11-docosénoïque) et érucique (cis-13-docosénoïque).

Ces divers acides sont issus des huiles végétales extraites de diverses plantes oléagineuses telles que le tournesol, le colza, le ricin, le lesquerella, l'olive, le soja, le palmier, l'avocat, l'argousier, la coriandre, le céleri, l'aneth, la carotte, le fenouil, le Limnanthes Alba (meadowfoam).
Ils sont issus également du monde animal terrestre ou marin, et dans ce dernier cas, aussi bien sous forme de poissons, de mammifères que d'algues. Il s'agit en général de graisses provenant de ruminants, de poissons comme la morue, ou de mammifères marins comme les baleines ou les dauphins.

L'invention vise un procédé de synthèse d'un ω-aminoacide(ester) de formule ROOC-(CH₂)_{q}-CH₂NH₂ dans laquelle R est H ou un radical alkyle comportant de 1 à 4 atomes de carbone et q un indice entier compris entre 2 et 13, à partir d'un acide(ester) gras mono-insaturé de formule R₁-CH=CH-(CH₂)ₚ-COOR₂ dans laquelle R₁ est H ou un radical hydrocarboné comprenant de 4 à 11 atomes de carbone et le cas échéant une fonction hydroxyle, R₂ est H ou un radical alkyle comportant de 1 à 4 atomes de carbone et p un indice entier compris entre 2 et 11, comportant une étape réactionnelle d'ammoniation conduisant à la transformation de la fonction carbonyle en fonction nitrile caractérisé en ce que :
dans un premier stade on transforme l'acide/ester gras insaturé en un nitriles ω-insaturé de formule CH₂=CH-(CH₂)ₚ-CN en deux étapes successives (d'ordre indifférent) éthénolyse et ammoniation puis,
dans un deuxième stade on transforme ce nitrile ω-insaturé en un nitrile acide/ester de formule
R₃OOC- [CH=CH]ₓ-(CH₂)ₚ-CN dans laquelle R₃ est H ou un radical alkyle comportant de 1 à 4 atomes de carbone et x est 0 ou 1, soit par coupure oxydante du nitrile ω-insaturé, soit par une réaction de métathèse croisée du nitrile ω-insaturé avec un acrylate dé formule CH₂=CH-COOR₃ et,
dans un troisième stade à hydrogéner le nitrile acide en ω-aminoacide(ester) de formule ROOC-(CH₂) _{q}-CH₂NH₂. Le processus réactionnel est alors le suivant.

### Premier stade :

R₁-CH=CH-(CH₂)ₚ-COOH + CH₂=CH₂ ⇔ CH₂=CH-(CH₂)ₚ-COOH + CH₂=CH-R₁

CH₂=CH-(CH₂)ₚ-COOH + NH₃ → CH₂=CH-(CH₂)ₚ-CN + 2 H₂O

ou en inversant l'ordre des réactions

R₁-CH=CH-(CH₂)ₚ-COOH + NH₃ → R₁-CH=CH-(CH₂)ₚ-CN +2 H₂O

R₁-CH=CH-(CH₂)ₚ-CN + CH₂=CH₂ ⇔ CH₂=CH-(CH₂)ₚ-CN + CH₂=CH-R₁

### Deuxième stade :

- première variante

   CH₂=CH-(CH₂)ₚ-CN + (coupure oxydante) → HOOC-(CH₂)ₚ-CN + HCHO/HCOOH
- seconde variante

   CH₂=CH-(CH₂)ₚ-CN + CH₂=CH-COOR₃ ⇔ R₃OOC-CH=CH-(CH₂)ₚ-CN + CH₂=CH₂

### Troisième stade :

première variante : HOOC-(CH₂)ₚ-CN +2 H₂ → HOOC-(CH₂)_{q}-CH₂NH₂
seconde variante : R₃OOC-CH=CH-(CH₂)ₚ-CN + 3 H₂→ R₃OOC-(CH₂)_{q}-CH₂NH₂,

Dans ce mode réalisation du procédé q est égal à p ou à p+2.
Appliqué à l'acide oléique le processus devient

### Premier stade :

CH₃-(CH₂)₇-CH=CH-(CH₂)₇-COOH + CH₂=CH₂ ⇔ CH₂=CH-(CH₂)₇-COOH + CH₂=CH-(CH₂)₇-CH₃

CH₂=CH-(CH₂)₇-COOH + NH₃ → CH₂=CH-(CH₂)₇-CN + 2 H₂O

ou en inversant l'ordre des réactions

CH₃-(CH₂)₇-CH=CH-(CH₂)₇-COOH + NH₃ → CH₃-(CH₂)₇-CH=CH-(CH₂)₇-CN +2 H₂O

CH₃-(CH₂)₇-CH=CH-(CH₂)₇-CN + CH₂=CH₂ ⇔ CH₂=CH-(CH₂)₇-CN + CH₂=CH-(CH₂)₇-CH₃

### Deuxième stade :

- première variante

   CH₂=CH-(CH₂)₇-CN + (coupure oxydante) → HOOC-(CH₂)₇-CN + HCHO/HCOOH
- seconde variante

   CH₂=CH-(CH₂)₇-CN + CH₂=CH-COOH ⇔ HOOC-CH=CH-(CH₂)₇ -CN + CH₂=CH₂

### Troisième stade :

première variante : HOOC-(CH₂)₇-CN +2 H₂ → HOOC-(CH₂)₇-CH₂NH₂
seconde variante : HOOC-CH=CH-(CH₂)₇-CN + 3 H₂→ HOOC-(CH₂)₉-CH₂NH₂

Les seuls « sous-produits » formés sont une α-oléfine à longue chaîne, comportant le cas échéant une fonction hydroxyle, et du formaldéhyde/acide formique.
Dans une variante simplifiée de réalisation du procédé de l'invention on peut gagner une étape en synthétisant lors du premier stade le nitrile de l'acide/ester gras de formule R₁-CH=CH-(CH₂)ₚ-CN par ammoniation de l'acide/ester d'origine puis en soumettant ce dernier à une métathèse croisée avec un acrylate R₃OOC-CH=CH₂ pour obtenir le nitrile-acide de formule R₃OOC-CH=CH-(CH₂)ₚ-CN qui sera ensuite hydrogéné en R₃OOC-(CH₂)ₚ₊₂-CH₂NH₂.

Dans une autre variante du procédé utilisant comme matière première les acides gras hydroxylés tels que l'acide ricinoléique et l'acide lesquérolique qui répondent à la formule générale R₁-CH=CH-(CH₂)ₚ-COOH avec R₁ égal à CH₃-(CH₂)₅CHOH-CH₂- et p égal respectivement 7 et 9 on soumet l'acide sous sa forme ester méthylique à une pyrolyse conduisant à un ester ω-insaturé de formule CH₂=CH-(CH₂)ₚ-₁-COOCH₃ qui est transformé, directement ou en passant par l'acide, en un nitrile ω-insaturé de même nature que celui du composé intermédiaire obtenu au terme du premier stade du procédé décrit ci-dessus. Cette variante consiste donc à remplacer pour ces acides gras particuliers l'éthénolyse initiale par une pyrolyse.
Les stades suivants du procédé sont analogues à ceux du procédé décrit ci-dessus. Ils conduisent donc à des composés de formules ROOC-(CH₂)_{q}-CH₂NH₂ dans laquelle q est égal à p+1 ou à p+3 selon la voie retenue lors du deuxième stade.

Ainsi, dans des modes de réalisations préférés de l'invention :
- lors du premier stade on effectue tout d'abord l'éthénolyse de l'acide (ester) pour la faire suivre par l'ammoniation de l'acide ω-alcénoïque ;
- lors du premier stade on effectue tout d'abord l'ammoniation de l'acide (ester) pour la faire suivre par l'éthénolyse du nitrile de l'acide gras de départ ;
- lors du premier stade on effectue tout d'abord la pyrolyse de l'acide (ester) gras hydroxylé pour la faire suivre par l'ammoniation de l'acide(ester) ω-alcénoïque issu de la pyrolyse ;
- lors du premier stade on effectue l'ammoniation de l'acide (ester) sans procéder à la réaction d'éthénolyse ;
- lors du deuxième stade on soumet le nitrile ω-insaturé de formule CH₂=CH-(CH₂)ₚ-CN à une coupure oxydante ;
- lors du deuxième stade on soumet le produit issu du premier stade à une réaction de métathèse croisée avec le composé de type acrylate ; et/ou
- le composé issu du deuxième stade est soumis à une hydrogénation.

Les réactions de métathèse sont connues depuis longtemps même si leurs applications industrielles sont relativement limitées. On peut se référer à propos de leur utilisation dans la transformation des acides (esters) gras, à l'article de J.C. Mol « Catalytic metathesis of unsaturated fatty acid esters and oil » paru Topics in Catalysis of unsaturated fatty esters and oil Vol. 27, Nos. 1-4, February 2004 (Plenum Publishing).
La catalyse de la réaction de métathèse a fait l'objet de très nombreux travaux et le développement de systèmes catalytiques sophistiqués. On peut citer par exemple les complexes au tungstène développés par Schrock et al (J. Am. Chem. Soc. 108 (1986) 2771 ou Basset et al. Angew. Chem., Ed. Engl. 31 (1992) 628. Plus récemment, sont apparus les catalyseurs dits de Grubbs (Grubbs et al. Angew. Chem., Ed. Engl. 34 (1995) 2039 et Organic Lett. 1 (1999) 953) qui sont des complexes ruthénium-benzylidène. Il s'agit de catalyse homogène. Il a été aussi développé des catalyseurs hétérogènes à base de métaux tels que Rhénium, Molybdène et Tungstène déposés sur alumine ou silice.

Enfin des travaux ont été conduits pour la réalisation de catalyseurs immobilisés, c'est-à-dire de catalyseurs dont le principe actif est celui du catalyseur homogène, notamment les complexes ruthénium-carbène, mais qui est immobilisé sur un support inactif. L'objectif de ces travaux est d'augmenter la sélectivité de la réaction de métathèse croisée vis-à-vis des réactions parasites telles que les « homo-métathèses » entre les réactifs mis en présence. Ils portent non seulement sur la structure des catalyseurs mais également sur l'incidence du milieu réactionnel et les additifs pouvant être introduits.
Dans le procédé de l'invention, tout catalyseur de métathèse actif et sélectif pourra être utilisé. On utilisera cependant de préférence des catalyseurs à base de ruthénium.
La réaction de métathèse croisée avec l'éthylène lors de l'une des étapes de la première phase est conduite à une température comprise entre 20 et 100°C à une pression de 1 à 30 bars en présence d'un catalyseur de métathèse classique par exemple de type Ruthénium. Le temps de réaction est choisi en fonction des réactifs mis en oeuvre et pour atteindre au plus près l'équilibre de la réaction. La réaction est effectuée sous une pression d'éthylène.

La réaction de métathèse croisée avec le composé de type acrylate est réalisée dans des conditions parfaitement connues. La température de réaction est comprise entre 20 et 100 °C généralement à la pression atmosphérique pour permettre un dégagement aisé de l'éthylène en présence d'un catalyseur à base de ruthénium.

Les catalyseurs au ruthénium sont choisis de préférence parmi les catalyseurs chargés ou non-chargés de formule générale :

(X1)a (X2)bRu(carbène C) (L1)c(L2)d

dans laquelle :
- a, b, c, d sont des nombres entiers avec a et b égaux à 0, 1 ou 2 ; c et d égaux à 0, 1, 2, 3 ou 4 ;
- X1 et X2, identiques ou différents, représentent chacun un ligand mono- ou multi-chélatant, chargé ou non ; à titre d'exemples, on pourra citer les halogénures, le sulfate, le carbonate, les carboxylates, les alcoolates, les phénolates, les amidures, le tosylate, l'hexafluorophosphate, le tétrafluoroborate, le bis-triflylamidure, le tétraphénylborate et dérivés. X1 ou X2 peuvent être liés à Y1 ou Y2 ou au (carbène C) de façon à former un ligand bidenté (ou chélate) sur le ruthénium ; et
- L1 et L2 identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéthers, ou un carbène hétérocyclique
L1 ou L2 peuvent être liés au "carbène C " de façon à former un ligand bidenté ou chélate,
Le "carbène C" pourra être représenté par la formule générale : C_(R1)_(R2) pour laquelle R1 et R2 sont identiques ou différents tels que l'hydrogène ou tout autre groupe hydrocarbonyle saturé ou insaturé, cyclique, branché ou linéaire, ou aromatique. A titre d'exemples, on pourra citer les complexes du ruthénium alkylidènes, ou cumulènes tels que les vinylidènes Ru=C=CHR ou allénylidènes Ru=C=C=CR1 R2 ou indénylidènes.
Un groupe fonctionnel permettant d'améliorer la rétention du complexe du ruthénium dans le liquide ionique peut être greffé sur au moins l'un des ligands X1, X2, L1, L2, ou sur le carbène C. Ce groupe fonctionnel peut être chargé ou non chargé tel que de préférence un ester, un éther, un thiol, un acide, un alcool, une amine, un hétérocycle azoté, un sulfonate, un carboxylate, un ammonium quaternaire, un guanidinium, un phosphonium quaternaire, un pyridinium, un imidazolium, un morpholinium ou un sulfonium.

Le schéma réactionnel de la synthèse des nitriles à partir des acides, bien connue de l'homme de l'art, peut se résumer de la façon suivante.

R-COOH + NH₃ → [R-COO⁻NH₄⁺] → [R-CONH₂] + H₂O → RCN + H₂O

Ce schéma s'applique tout aussi bien aux acides (esters) gras naturels qu'aux acides gras ω-insaturés.
Le procédé peut être conduit en batch en phase liquide ou gazeuse ou en continu en phase gazeuse. La réaction est conduite à température élevée > 250 °C et présence d'un catalyseur qui est généralement un oxyde métallique et le plus fréquemment l'oxyde de zinc. L'élimination en continu de l'eau formée en entraînant de surcroît l'ammoniac qui n'a pas réagi permet un achèvement rapide de la réaction.
La réaction de pyrolyse mise en oeuvre dans la variante du procédé est réalisée sur la forme ester de l'acide gras hydroxylé concerné en général le méthylate. La réaction est conduite à haute température, comprise entre 400 et 750 °C et de préférence entre 500 et 600 °C en présence de vapeur d'eau surchauffée.

La réaction de pyrolyse appliquée au ricinoléate de méthyle répond au processus suivant :

CH₃-(CH₂)₅CHOH-CH₂-CH=CH-(CH₂)₇-COOCH₃ + Δ → CH₃-(CH₂)₅CHO + CH₂=CH-(CH₂)₇-COOCH₃

Elle est suivie par une ammoniation :

CH₂=CH-(CH₂)₇-COOCH₃ + NH₃ → CH₂=CH-(CH₂)₇-CN + 2 H₂O.

L'étape de synthèse des ω-aminoacides(esters) gras à partir des nitrile-acides gras consiste en une hydrogénation classique. Les catalyseurs sont nombreux mais préférentiellement on utilise les Nickel et Cobalt de Raney. Afin de favoriser la formation de l'amine primaire, on opère avec une pression partielle d'ammoniac. Enfin, la réduction de la fonction nitrile en amine primaire est bien connue de l'homme de l'art.
La réaction de coupure oxydante de la double liaison, qui conduit à la formation de la fonction acide sur les deux carbones de la double liaison, est également en elle-même connue. Elle peut être réalisée en utilisant une large gamme d'agents oxydants forts.

Elle peut par exemple être réalisée au moyen d'un oxydant fort tel que KMnO₄ sous forme concentrée et à la chaleur ainsi que cela est décrit dans « Organic Chemistry » de L.G. Wade Jr. 5th Edition Chapter 8 Reactions of Alkenes.
La coupure oxydante peut être obtenue par une voie sulfochromique telle que décrite dans le brevet USP 2,871,247 aux colonnes 2 et 3.
D'autre part l'article de G.S. Zhang et al. dans Chinese Chemical Letters, Vol 5, N°2, pp105-108 de 1994, indique qu'il est possible d'effectuer la coupure oxydante à partir du diol correspondant à l'acide oléique (voir Entrée 29 du tableau) . Cette coupure oxydante est effectuée en utilisant le chlorochromate d'ammonium comme oxydant. Le diol quant à lui est obtenu par époxydation de l'acide oléique suivie d'une hydrolyse du pont époxy.
L'article de F. Drawert et al. dans Chem. Mikrobiol. Technol. Lebensm. 1, 158-159 (1972) décrit une voie alternative par irradiation de l'huile de tournesol.
La coupure oxydante peut être effectuée avec de l'eau oxygénée comme décrit dans le brevet GB 743,491. L'utilisation de H₂O₂ est également décrite dans le brevet WO07039481 (Novamont).
On peut également mentionner l'ouvrage Angew. Chem. Int. Ed. 2000,39, pp. 2206-2224 qui décrit la coupure oxydante de la double liaison soit avec un peracide associé à un catalyseur à base de ruthénium, soit avec H₂O₂ avec des catalyseurs à base de Mo, W ou Re.

De nombreux travaux ont été conduits sur l'utilisation de l'ozone comme agent oxydant. Il est d'ailleurs mentionné dans l'ouvrage Angew. Chem. cité ci-dessus que la coupure oxydante de l'acide oléique en acides pélargonique et azélaique est la plus importante application industrielle de l'ozonolyse
Le brevet USP 2,813,113 notamment décrit un procédé d'ozonolyse oxydante d'un acide gras tel que l'acide oléique qui consiste dans une première étape à traiter l'acide par de l'oxygène associé à de l'ozone pour former des ozonides puis dans une deuxième étape à oxyder ces derniers par l'oxygène.

Dans ce type de réaction on n'utilise pas de composés bloquant le processus l'oxydation au stade des cétones ou aldéhydes dans ce qui est appelé l'ozonolyse réductrice qui a fait plus récemment l'objet de travaux importants.

Dans le procédé de l'invention, on peut traiter l'acide gras soit sous sa forme acide, soit sous sa forme ester. Le passage d'une forme à l'autre, par méthanolyse, estérification ou hydrolyse, parfaitement banal ne constitue pas une transformation chimique au sens du procédé.

Tous les mécanismes détaillés ci-dessous illustrent, pour faciliter l'exposé, la synthèse des acides. Cependant, la métathèse est aussi efficace avec un ester et même plus efficace, le milieu étant généralement plus anhydre. De la même manière, les schémas illustrent des réactions avec l'isomère cis des acides (ou esters) ; les mécanismes sont aussi bien applicables aux isomères trans.

Le mécanisme réactionnel de cette réaction est illustrée par le schéma 1 ci-dessous 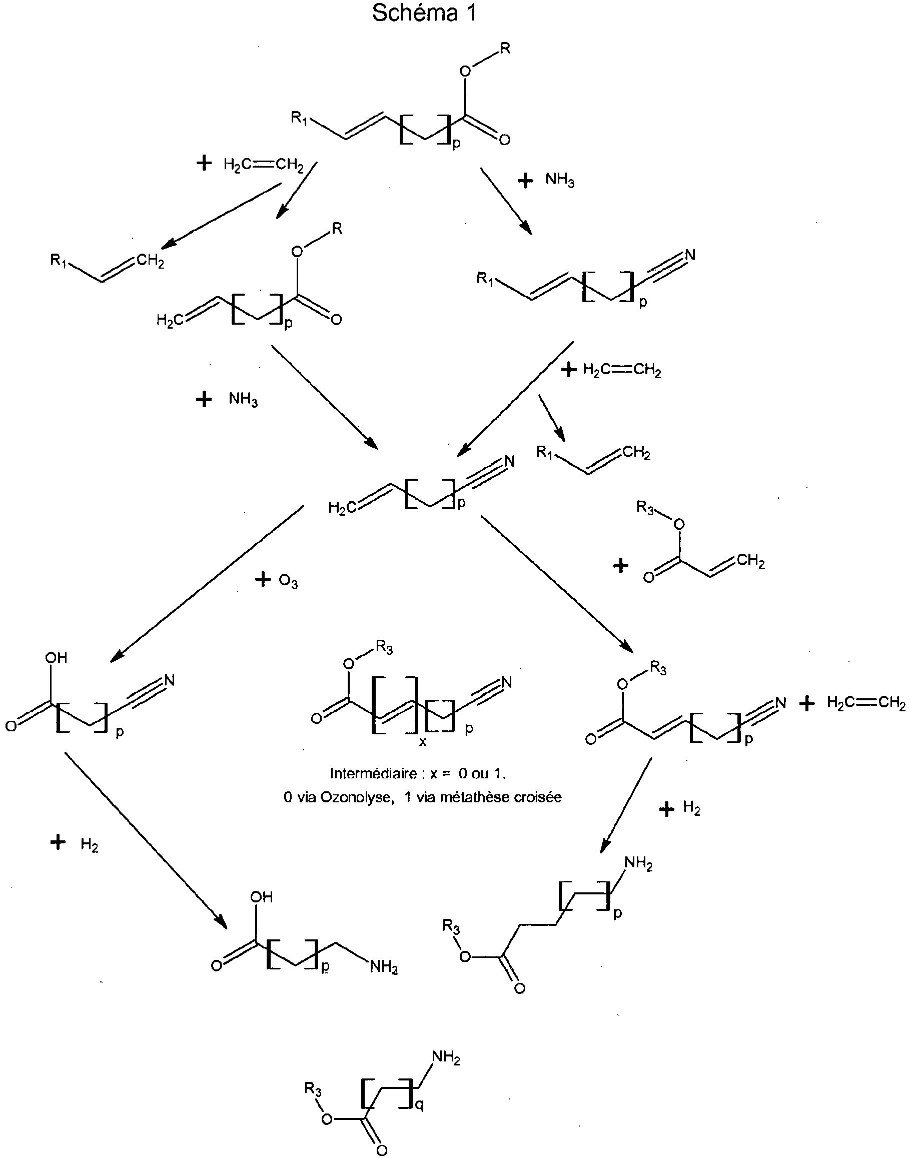 (dans le schéma ci-dessus q=p selon la voie ozonolyse, et q=p+2 selon la voie de métathèse croisée)
La variante de mise en oeuvre du procédé de l'invention appliquée aux acides gras insaturés hydroxylés est illustrée par le schéma 2 ci-dessous.

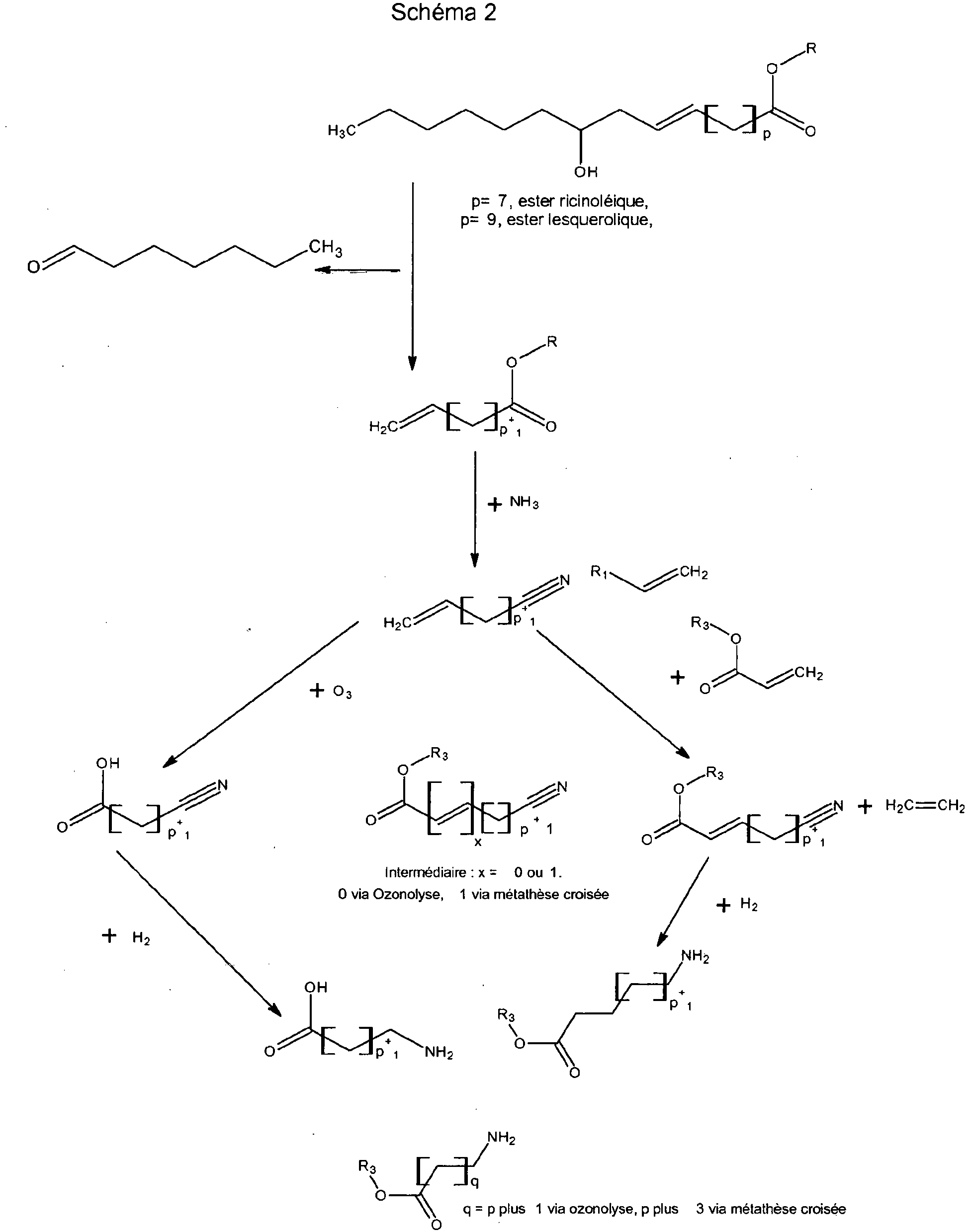

L'invention concerne en outre l'aminoacide ou aminoester d'origine renouvelable de formule générale NH₂CH₂-(CH₂)_{q}-COOR, R étant soit H, soit un radical alkyle comportant de 1 à 4 atomes de carbone.
Par aminoacides ou aminoesters d'origine renouvelable, on entend les aminoacides ou aminoesters qui comprennent du carbone d'origine renouvelable.

En mettant en oeuvre le procédé de l'invention, il sera possible de synthétiser toute une gamme d'ω-aminoacides.
L'acide 4-aminotétranoïque est obtenu à partir des acides obtusilique et lindérique et tsuzuique .
L'acide 5-aminopentanoïque est obtenu à partir de l'acide lauroléique, physétérique et cis-5-eicosénoïque.
L'acide 6-aminohexanoïque est obtenu à partir des acides obtusilique, lindérique, tsuzuique et petrosélinique.
L'acide 7-aminoheptanoïque est obtenu à partir de l'acide lauroléique, physétérique, cis 5 eicosénoïque.
L'acide 8-aminooctanoïque est obtenu à partir de l'acide petrosélinique.
L'acide 9-aminononanoïque est obtenu à partir des acides caproléique, myristoléique, palmitoléique, oléique, élaidique, ricinoléique et gadoléique,
L'acide 10-aminodécylénique est obtenu à partir de l'acide ricinoléique
L'acide 11-aminoundécylénique est obtenu à partir des acides caproléique, myristoléique, palmitoléique, oléique, élaidique, ricinoléique, gadoléique, vaccénique, gondoïque, lesquérolique et cétoléique.
L'acide 12-aminododécylénique est obtenu à partir des acides ricinoléique et lesquérolique.
L'acide 13-aminotridécylénique est obtenu à partir des acides vaccénique, gondoïque, cétoléique, lesquérolique et érucique.
L'acide 14-aminotétradécylénique est obtenu à partir de l'acide lesquérolique.
L'acide 15-aminopentadécylénique est obtenu à partir de l'acide érucique.

L'invention est illustrée par les exemples suivants.

### Exemple 1

Cet exemple illustre la première étape d'éthénolyse de l'oléate de méthyle selon le procédé objet de l'invention. On utilise pour cette réaction le catalyseur complexe [RuCl₂(=CHPh)(IMesH₂)(PCy₃)] dont la formule (A) est donnée ci-dessous. On effectue la réaction dans CH₂Cl₂, à une concentration de 0,05 M d'oléate de méthyle et 0,2 M d'éthylène à une température de 55°C à la pression atmosphérique, et pendant 6 heures, en présence du catalyseur à une concentration de 5 % molaire par rapport à l'oléate de méthyle. Les rendements sont déterminés par analyse chromatographique. On peut mesurer un rendement en 9-décénoate de méthyle CH₂=CH-(CH₂)₇-COOCH₃ et en 1-décène de 55 % molaire.

### Catalyseur de formule (A)

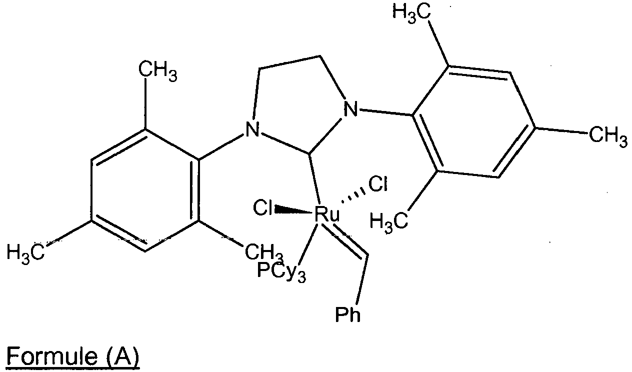

### Exemple 2

Cet exemple illustre la deuxième étape d'ammoniation transformant l'acide 9-décénoïque, issu après hydrolyse de l'ester de la première étape, en nitrile de formule CN-(CH₂)₇- CH=CH₂.
La réaction d'ammoniation de l'acide 9-décénoïque (3,5g) pour former le nitrile ω-insaturé de formule CN-(CH₂)₇-CH=CH₂ est conduite en batch avec introduction d'ammoniac en excès molaire par rapport à l'acide et à une température de 300°C à la pression atmosphérique (en phase gazeuse), en présence d'un catalyseur d'oxyde de zinc. Le réacteur est équipé d'un condenseur à 100 °C. De l'ammoniac est aussi injectée en continu pendant 6 heures. L'élimination en continu de l'eau formée entraîne l'ammoniac en excès permet un achèvement rapide de la réaction. On récupère 2,6 g du nitrile qui sont séparés par distillation sous vide.

### Exemple 3

Cet exemple illustre la coupure oxydante du nitrile ω-insaturé issu de l'étape de l'exemple 2 par ozonolyse pour former le nitrile acide de formule CN-(CH₂)₇-COOH.

De l'ozone obtenue par un générateur d'ozone Welsbach T-408, est bullée dans 25 ml de pentane jusqu'à ce qu'une couleur bleue soit observée. La solution de pentane est maintenue à -70°C avec un bain d'acétone-carboglace. 20 mg d'ester méthylique d'un nitrile obtenu conformément à l'exemple 2, dissous dans 5 ml de pentane refroidis à 0 °C sont ajoutés à la solution d'ozone. L'excès d'ozone est ensuite éliminé et la couleur bleue disparaît. Après 5 minutes, le pentane est évaporé par un flux d'azote sec. Pendant cette étape la température de la solution est maintenue inférieure à 0°C. Après évaporation du pentane, 3 ml de méthanol refroidis à -70 °C sont ajoutés au réacteur, tout en le réchauffant pour permettre la dissolution de l'ozonide. Pour effectuer la conversion de l'ozonide en acide-nitrile, on commence par élever la température à environ 60 °C. Quand la réaction de décomposition de l'ozonide démarre, elle s'accompagne d'une élévation de la température. On ajoute en continu un flux d'oxygène, pour maintenir la température et pour oxyder directement les produits issus de la décomposition de l'ozonide. On procède en 4 heures pour limiter la formation de produits de dégradation. Il est important de maintenir la température de réaction légèrement au-dessus de la température de décomposition de l'ozonide au cours de cette étape. Une température de 95 °C est utilisée dans cet exemple.
On obtient 6 mg de nitrile acide de formule CN-(CH₂)₇-COOH.

### Exemple 3 bis

Cet exemple illustre une variante de coupure oxydante du nitrile ω-insaturé.

50 g d'un nitrile insaturé synthétisés conformément à l'exemple 2 sont ozonisés à - 40 °C dans du chlorure d'éthyle en utilisant de l'oxygène contenant 3,7 % d'ozone. Le solvant est ensuite distillé et l'ozonide est traité sous reflux pendant 30 minutes avec 100 g d'eau. Le mélange est ensuite refroidi, on ajoute du carbonate de sodium en excès et le mélange est agité à 40 °C pendant 10 minutes. La fraction non dissoute est séparée et éliminée. La fraction soluble est acidifiée avec de l'acide chlorhydrique à 10 % et les acides sont séparés et séchés sur du sulfate de magnésium. Le mélange d'aldéhydes est oxydé par de l'oxygène moléculaire pendant 1 heure à 120-140 °C en présence d'oxyde ferrique. Les acides sont extraits avec une solution de carbonate de sodium et libérés par une solution d'acide chlorhydrique, séparés et séchés sur sulfate de magnésium anhydre. Les deux lots d'acides sont combinés et distillés sous vide à 196 °C (5 mm Hg).
15 g de nitrile acide sont dissous dans 160 g d'éthanol et 15 g d'ammoniac liquide, La solution est placée dans un autoclave agité, avec 3 g de catalyseur nickel de Raney, et sous une pression de 110 bars d'hydrogène. La température est élevée à 100 °C, et la pression augmente jusqu'à 139 bars. Les conditions sont maintenues pendant 4 heures. L'autoclave est refroidi et le contenu est filtré pour récupérer le catalyseur. 50 g d'eau sont alors ajoutés et l'alcool est distillé. La solution résultante est titrée avec de l'acide chlorhydrique dilué et l'acide aminononanoïque est filtré , lavé et traité sous reflux d'acétone et séché.

### Exemple 4

Cet exemple illustre la réaction de métathèse croisée entre un nitrile de formule CN-(CH₂)₇-CH=CH₂ issu de l'étape de l'exemple 2 avec l'acrylate de méthyle selon la réaction :

CH₂=CH-(CH₂)₇-CN + CH₂=CH-COOCH₃ ⇔ CH₃OOC-CH=CH-(CH₂)₇ -CN + CH₂=CH₂

Dans un tube de Schlenk de 50 ml, on charge 83 mg de 9-cyanoundécène (0,5 mmol), 86 mg d'acrylate de méthyle (1 mmol) et 10 ml de toluène distillé sur sodium-benzophénone. On rajoute 1,5 mg (2,4.10⁻³ mmol) de catalyseur de Hoveyda-Grubbs de seconde génération [(1,3-bis(2,4,6-triméthylphényl)-2-imidazolidinylidène)dichloro(o-isopropoxyphénylméthylène] ruthénium, commercialisé par la société Aldrich®). Sous azote et sous agitation magnétique, on chauffe à 100°C et on laisse réagir 1 heure. Le mélange réactionnel est analysé par chromatographie en phase gaz (étalon dodécane). La conversion est de 70%. La sélectivité en nitrile ester de méthyle (mélange cis + trans) est de 100%.

### Exemple 5

Cet exemple illustre la variante avec inversion de l'ordre des 2 étapes de la phase 1: ammoniation de l'acide gras insaturé puis éthénolyse du nitrile insaturé.

L'ammoniation l'acide oléique est conduite en batch avec introduction d'ammoniac en excès molaire par rapport à l'acide et à une température de 300°C à la pression atmosphérique (en phase gazeuse), en présence d'un catalyseur d'oxyde de zinc. L'élimination en continu de l'eau formée entraîne l'ammoniac en excès permet un achèvement rapide de la réaction.

L'éthénolyse du nitrile de l'acide oléique est conduite à 60°C à la pression atmosphérique en présence d'un catalyseur à base de Ruthénium [RuCl₂(=CHPh)(IMesH₂)(PCy₃)] en utilisant un excès d'éthylène pour obtenir l'acide 9-décénoïque CH₂=CH-(CH₂)₇-COOH. Les rendements sont déterminés par analyse chromatographique. Au terme de la réaction, 6 heures, on sépare l'α-oléfine en C10 par distillation sous vide pour obtenir le nitrile 9-décénoïque CH₂=CH-(CH₂)₇-CN. Les rendements sont déterminés par analyse chromatographique. On peut mesurer un rendement de 55 %.

### Exemple 6

### Pyrolyse d'acide gras hydroxylé.

Le triglycéride de l'acide ricinoléique est transestérifié par un excès de méthanol en présence de méthylate de sodium.
L'ester est alors vaporisé à 225 °C et ensuite mélangé avec de la vapeur d'eau surchauffée (620°C). La réaction est brève, une dizaine de secondes environ. On procède ensuite à la purification de l'undécénoate de méthyle, tout d'abord par refroidissement du milieu qui permet l'extraction de l'eau puis par une série de distillations permettant la séparation de l'ester et des sous-produits de la réaction.

### Exemple 7

### Hydrogénation

L'hydrogénation de la double liaison et de la fonction nitrile est réalisée en présence d'un catalyseur constitué d'un nickel de Raney

1 g de nitrile acide de formule CN-(CH₂)₇-COOH obtenu conformément à l'exemple 3 est estérifié avec le méthanol. On introduit dans un réacteur 1g de l'acide-nitrile, 1,2 g de méthanol, 1,2 g de benzène et quelques gouttes d'acide sulfurique concentré. L'azéotrope eau-alcool-benzène est éliminé en tête de colonne. De l'acide sulfurique est ajouté en continu pour maintenir l'avancement de la réaction. Ensuite le benzène et l'alcool sont flashés pour récupérer le nitrile ester : 1,02 g.

Le nitrile-ester synthétisé est placé dans un autoclave agité de 15 ml et on y ajoute 2,5 g d'éthanol à 96 %, 2,5 g d'ammoniac liquide et 0,125 g de catalyseur nickel de Raney contenant 3 % poids de cobalt. Le mélange est chauffé à 90 °C, sous 150 bars d'hydrogène (pression totale 210 bars) et pendant 4 heures. L'ester méthylique est distillé sous vide de 0,5 mm de mercure. Un distillat clair de 0,97 g est récupéré. Il contient 90 % d'amino ester.

### Exemple 8

L'aminoacide est destiné à être polymérisé. Pour cela on procède à une hydrolyse de l'amino ester. L'amino-9-nonanoate de méthyle obtenu est placé dans une ampoule à brome pour tomber goutte à goutte dans un ballon tricol de 2 litres surmonté d'une longue colonne à distiller et contenant un litre d'eau bouillante. Le reflux est réglé de façon à distiller le méthanol formé, ce qui permet de suivre la réaction, l'hydrolyse dure 4 à 5 heures pour l'ester méthylique. Lorsque la réaction est terminée, on filtre à chaud et évapore l'eau. On obtient un produit difficile à sécher au dessiccateur alors qu'en lavant le produit humide à l'acétone et en le séchant au dessiccateur, on recueille 20 g d'acide aminé brut incolore.

## Revendications

1. Procédé de synthèse d'un ω-aminoacide(ester) de formule ROOC-(CH₂)_{q}-CH₂NH₂ dans laquelle R est H ou un radical alkyle comportant de 1 à 4atomes de carbone et q un indice entier compris entre 2 et 13, à partir d'un acide(ester) gras mono-insaturé de formule R₁-CH=CH-(CH₂)ₚCOOR₂ dans laquelle R₁ est H ou un radical hydrocarboné comprenant de 4 à 11 atomes de carbone et le cas échéant une fonction hydroxyle, R₂ est H ou un radical alkyle comportant de 2 à 4 atomes de carbone et p un indice entier compris entre 2 et 11 ,
comportant une étape réactionnelle d'ammoniation conduisant à la transformation de la fonction carbonyle en fonction nitrile **caractérisé en ce que** dans un premier stade on transforme l'acide/ester gras insaturé en un nitrile ω-insaturé de formule CH₂=CH-(CH₂)ₚ-CN en deux étapes successives (d'ordre indifférent) éthénolyse et ammoniation puis,
dans un deuxième stade on transforme ce nitrile ω-insaturé en un nitrile acide de formule
R₃OOC- [CH=CH]ₓ-(CH₂)ₚ-CN dans laquelle R₃ est H ou un radical alkyle comportant de 1 à 4 atomes de carbone, x est 0 ou 1, soit par coupure oxydante du nitrile ω-insaturé, soit par une réaction de métathèse croisée du nitrile ω-insaturé avec un acrylate de formule CH₂=CH-COOR₃ et,
dans un troisième stade à hydrogéner le nitrile acide en ω-aminoacide(ester) de formule ROOC-(CH₂)_{q}-CH₂NH₂.

2. Procédé selon la revendication 1 **caractérisé en ce que** lors du premier stade on effectue tout d'abord l'éthénolyse de l'acide (ester) pour la faire suivre par l'ammoniation de l'acide ω-alcénoïque.

3. Procédé selon la revendication 1 **caractérisé en ce que** lors du premier stade on effectue tout d'abord l'ammoniation de l'acide (ester) pour la faire suivre par l'éthénolyse du nitrile de l'acide gras de départ.

4. Procédé selon la revendication 1 **caractérisé en ce que** lors du premier stade on effectue tout d'abord la pyrolyse de l'acide (ester) gras hydroxylé pour la faire suivre par l'ammoniation de l'acide(ester) ω-alcénoïque issu de la pyrolyse.

5. Procédé selon la revendication 1 **caractérisé en ce que** lors du premier stade on effectue l'ammoniation de l'acide (ester) sans procéder à la réaction d'éthénolyse.

6. Procédé selon l'une des revendications 1 à 4 **caractérisé en ce que** lors du deuxième stade on soumet le nitrile ω-insaturé de formule CH₂=CH-(CH₂)ₚ-CN à une coupure oxydante.

7. Procédé selon l'une des revendications 1 à 5 **caractérisé en ce que** lors du deuxième stade on soumet le produit issu du premier stade à une réaction de métathèse croisée avec le composé de type acrylate.

8. Procédé selon l'une des revendications 1 à 7 **caractérisé en ce que** le composé issu du deuxième stade est soumis à une hydrogénation.

## Patentansprüche

1. Verfahren zur Synthese einer ω-Aminosäure bzw. eines ω-Aminosäureesters der Formel ROOC-(CH₂)_{q}-CH₂NH₂, worin R für H oder einen Alkylrest mit 1 bis 4 Kohlenstoffatomen steht und q für eine ganze Zahl zwischen 2 und 13 steht, aus einer einfach ungesättigten Fettsäure bzw. einem einfach ungesättigten Fettsäureester der Formel R₁-CH=CH-(CH₂)ₚ-COOR₂, worin R₁ für H oder einen Kohlenwasserstoffrest mit 4 bis 11 Kohlenstoffatomen und gegebenenfalls einer Hydroxylfunktion steht, R₂ für H oder einen Alkylrest mit 2 bis 4 Kohlenstoffatomen steht und p für eine ganze Zahl zwischen 2 und 11 steht, umfassend einen Ammonierungsreaktionsschritt, der zur Umwandlung der Carbonylfunktion in eine Nitrilfunktion führt, **dadurch gekennzeichnet, dass** man in einer ersten Stufe die ungesättigte Fettsäure bzw. den ungesättigten Fettsäureester in zwei aufeinanderfolgenden Ethenolyse- und Ammonierungsschritten (in beliebiger Reihenfolge) in ein ω-ungesättigtes Nitril der Formel CH₂=CH-(CH₂)ₚ-CN umwandelt, dann
in einer zweiten Stufe dieses ω-ungesättigte Nitril entweder durch oxidative Spaltung des ω-ungesättigten Nitrils oder durch eine Kreuzmetathesereaktion des ω-ungesättigten Nitrils mit einem Acrylat der Formel CH₂=CH-COOR₃ in eine Nitrilsäure der Formel R₃OOC-[CH=CH]ₓ-(CH₂)ₚ-CN, worin R₃ für H oder einen Alkylrest mit 1 bis 4 Kohlenstoffatomen steht und x 0 oder 1 bedeutet, und
in einer dritten Stufe die Nitrilsäure zu der ω-Aminosäure bzw. zu dem ω-Aminosäureester der Formel ROOC-(CH₂)_{q}-CH₂NH₂ hydriert.

2. Verfahren nach Anspruch 1, **dadurch gekennzeichnet, dass** man in der ersten Stufe zunächst die Ethenolyse der Säure bzw. des Esters durchführt und darauf die Ammonierung der ω-Alkensäure folgen lässt.

3. Verfahren nach Anspruch 1, **dadurch gekennzeichnet, dass** man in der ersten Stufe zunächst die Ammonierung der Säure bzw. des Esters durchführt und darauf die Ethenolyse des Nitrils der Ausgangsfettsäure folgen lässt.

4. Verfahren nach Anspruch 1, **dadurch gekennzeichnet, dass** man in der ersten Stufe zunächst die hydroxylierte Fettsäure bzw. den hydroxylierten Fettsäureester pyrolysiert und darauf die Ammonierung der aus der Pyrolyse stammenden ω-Alkensäure bzw. des aus der Pyrolyse stammenden ω-Alkensäureesters folgen lässt.

5. Verfahren nach Anspruch 1, **dadurch gekennzeichnet, dass** man in der ersten Stufe die Ammonierung der Säure bzw. des Esters durchführt, ohne zur Ethenolysereaktion überzugehen.

6. Verfahren nach einem der Ansprüche 1 bis 4, **dadurch gekennzeichnet, dass** man in der zweiten Stufe das ω-ungesättigte Nitril der Formel CH₂=CH-(CH₂)ₚ-CN einer oxidativen Spaltung unterwirft.

7. Verfahren nach einem der Ansprüche 1 bis 5, **dadurch gekennzeichnet, dass** man in der zweiten Stufe das aus der ersten Stufe stammende Produkt einer Kreuzmetathesereaktion mit der Verbindung vom Acrylat-Typ unterwirft.

8. Verfahren nach einem der Ansprüche 1 bis 7, **dadurch gekennzeichnet, dass** man die aus der zweiten Stufe stammende Verbindung einer Hydrierung unterwirft.

## Claims

1. A process for the synthesis of an ω-amino acid (ester) of formula ROOC-(CH₂)_{q}-CH₂NH₂, in which R is H or an alkyl radical comprising from 1 to 4 carbon atoms and q is an integral index of between 2 and 13, starting from a monounsaturated fatty acid (ester) of formula R₁-CH=CH-(CH₂)ₚ-COOR₂, in which R₁ is H or a hydrocarbon radical comprising from 4 to 11 carbon atoms and, if appropriate, a hydroxyl functional group, R₂ is H or an alkyl radical comprising from 2 to 4 carbon atoms and p is an integral index of between 2 and 11, comprising an ammoniation reaction stage resulting in the conversion of the carbonyl functional group to a nitrile functional group, **characterized in that**:
in a first stage, the unsaturated fatty acid/ester is converted to an ω-unsaturated nitrile of formula CH₂=CH-(CH₂)ₚ-CN in two successive (in any order) ethenolysis and ammoniation stages, then,
in a second stage, this ω-unsaturated nitrile is converted to an acid/ester nitrile of formula R₃OOC-[CH=CH]ₓ-(CH₂)ₚ-CN, in which R₃ is H or an alkyl radical comprising from 1 to 4 carbon atoms and x is 0 or 1, either by oxidative cleavage of the ω-unsaturated nitrile or by a cross metathesis reaction of the ω-unsaturated nitrile with an acrylate of formula CH₂=CH-COOR₃, and,
in a third stage, the acid/ester nitrile is hydrogenated to give an ω-amino acid (ester) of formula ROOC-(CH₂)_{q}-CH₂NH₂.

2. The process as claimed in claim 1, **characterized in that**, during the first stage, the ethenolysis of the acid (ester) is first of all carried out, to be followed by the ammoniation of the ω-alkenoic acid.

3. The process as claimed in claim 1, **characterized in that**, during the first stage, the ammoniation of the acid (ester) is first of all carried out, to be followed by the ethenolysis of the nitrile of the starting fatty acid.

4. The process as claimed in claim 1, **characterized in that**, during the first stage, the pyrolysis of the hydroxylated fatty acid (ester) is first of all carried out, to be followed by the ammoniation of the ω-alkenoic acid (ester) resulting from the pyrolysis.

5. The process as claimed in claim 1, **characterized in that**, during the first stage, the ammoniation of the acid (ester) is carried out, without proceeding to the ethenolysis reaction.

6. The process as claimed in one of claims 1 to 4, **characterized in that**, during the second stage, the ω-unsaturated nitrile of formula CH₂=CH-(CH₂)ₚ-CN is subjected to an oxidative cleavage.

7. The process as claimed in one of claims 1 to 5, **characterized in that**, during the second stage, the product resulting from the first stage is subjected to a cross metathesis reaction with the compound of acrylate type.

8. The process as claimed in one of claims 1 to 7, **characterized in that** the compound resulting from the second stage is subjected to a hydrogenation.
